# EUROPEAN PATENT SPECIFICATION

(11) **EP 4 552 551 B1**
(45) Date of publication and mention of the grant of the patent: **29.07.2026**
(21) Application number: 23835623.2
(22) Date of filing: 07.07.2023
(51) Int. Cl.: A61B 5/251, A61B 5/00, A61B 5/11, A61B 5/024

(54) **CONTACT MOTION DETERMINING MODULE AND BIOLOGICAL CONTACT DETECTION DEVICE USING SAME**
KONTAKTBEWEGUNGSBESTIMMUNGSMODUL UND LEBENDKÖRPERKONTAKTDETEKTIONSVORRICHTUNG DAMIT
MODULE DE DÉTERMINATION DE MOUVEMENT DE CONTACT ET DISPOSITIF DE DÉTECTION DE CONTACT AVEC UN CORPS VIVANT L'UTILISANT

(30) Priority: 08.07.2022 JP 2022110849
(43) Date of publication of application: 14.05.2025
(73) Proprietor: National University Corporation Chiba University, Chiba-shi, Chiba 263-8522 (JP); I Medex Co., Ltd., Chiba-shi, Chiba 262-0003 (JP)
(72) Inventor: YAMASHITA AMEMIYA, Ayumi, Chiba-shi, Chiba 260-8672 (JP); MATSUMURA, Aya, Chiba-shi, Chiba 260-8672 (JP); ICHIDA, Makoto, Chiba-shi, Chiba 262-0003 (JP); MINOWA, Takashiro, Chiba-shi, Chiba 262-0003 (JP)
(74) Representative: Hasegawa, Kan
(86) International application number: PCT/JP2023/025327
(87) International publication number: WO 2024/010092

(56) References cited:
- JP-A- 2007 020 801
- JP-A- 2022 015 347
- US-A1- 2019 232 111

## Description

### Technical Field

The present invention relates to a system for predicting a contact motion such as a wearer himself/herself removing a medical catheter or removing a bandage, a covering material, or the like, and a biological contact detection device using the system, and more particularly, to a contact motion determining module for predicting a contact by the wearer for removing or peeling, and the biological contact detection device using the contact motion determining module.

### Background Art

In a medical facility such as a hospital, a medical catheter is placed in a patient for medical treatment at the time of extracorporeal circulation in artificial dialysis, supply of a medicinal solution by drip infusion, and the like. In addition, bandages, covering materials (including "skin bonding tape", the same applies hereinafter), etc. are used to protect wounds for post-surgical patients, etc. However, there are various patients who receive such medical treatment, and for a patient with severe and mild consciousness disorder, a patient with dementia, or a patient with insufficient understanding of a therapeutic instrument or a use method, the patient may remove the medical catheter or remove a bandage, a covering material, or the like.

Therefore, in the past, some techniques for detecting the removal of the medical catheter have been proposed. For example, Patent Literature 1 (JP 2007-20801 A) proposes a monitoring device for a puncture site that predicts removal of a puncture needle due to a patient's own needle removal to avoid adverse effects caused by the removal of the puncture needle. The monitoring device for the puncture site includes a puncture needle capable of puncturing a patient, and a flexible tube which extends from the puncture needle and is capable of introducing liquid into the body of the patient or extracting blood from the body of the patient through the puncture needle. The monitoring device for the puncture site is configured to include: a detection means that is formed on a surface different from a contact surface of a fixing site which fixes the flexible tube in contact with the patient during medical treatment, and detects that the patient has been in contact with the fixing site; and a signal generation unit that generates a signal when the contact with the fixing site by the detection means is detected.

Furthermore, in the past, a technique for detecting a sign leading to removal of the drip needle before the drip needle is removed has also been proposed. For example, Patent Literature 2 (JP 2015-66071 A) proposes a monitoring device including: a first detection unit that detects a state of a place where a drip needle is stuck in a monitored target person based on an image obtained by photographing the monitored target person; a second detection unit that detects a state of a tube connected to the drip needle based on the image; a determination unit that determines whether there is a possibility of removal of the drip needle based on a change in at least one of the detected state of the place where the drip needle is stuck and the detected state of the tube; and an output unit that outputs an alarm indicating the possibility of removal of the drip needle when it is determined that there is the possibility of removal of the drip needle.

### Citation List

### Patent Literature

Patent Literature 1: JP 2007-20801 A
Patent Literature 2: JP 2015-66071 A

### Summary of Invention

### Technical Problem

The monitoring device for the puncture site according to Patent Literature 1 is configured such that the detection means detects that the patient has been in contact with the fixing site, and a signal generation unit generates a signal in response to the detection. However, since the generation of the signal is determined only by the detection of the contact, there is a problem of so-called false alarm in which the signal is generated even in response to a contact irrelevant to the removal of the puncture needle.

According to Patent Literature 2, the possibility of removal is predicted based on the state of the place where the drip needle is stuck and the state of the tube, but the state of the tube connected to the drip needle is detected based on an image obtained by photographing the monitored target person. For this reason, it is necessary to photograph images of the state of the patient who is the monitored target person, which not only raises privacy issues but also makes monitoring itself impossible when the site being imaged is in a blind spot.

Therefore, an object of the present invention is to provide a contact motion determining module capable of predicting and detecting an action of removing a medical catheter or removing a bandage, a covering material, a skin bonding tape, or the like by a wearer himself/herself such as a patient accurately without depending on a photographed image, and a biological contact detection device using the contact motion determining module.

Furthermore, another object of the present invention is to provide a contact motion determining module that can eliminate notification and the like at the time of other actions (that is, "false alarm") by identifying only an action of removing a medical catheter or removing a bandage, a covering material, a skin bonding tape, or the like among actions of a wearer himself/herself such as a patient, and a biological contact detection device using the contact motion determining module.

Another object of the present invention is to provide a sensor and a computer program for solving at least any one of the above problems.

### Solution to Problem

The problem is solved by the teachings of the independent claims. Further embodiments are defined in the dependent claims. The invention is defined by the appended claims.

The present disclosure provides a contact motion determining module that acquires a detection signal from a sensor that detects presence or absence of contact of a biological, and determines whether or not an action of removing a medical catheter or an action of removing a bandage, a covering material, a skin bonding tape, or the like (hereinafter referred to as "specific action") is performed based on the detection signal. In addition, a biological contact detection device using the contact motion determining module is provided.

That is, the present disclosure provides a contact motion determining module that predicts and determines a specific action of a wearer, the contact motion determining module including: a contact signal acquiring unit that acquires a contact signal from a contact detection sensor which detects a contact of the wearer; an action determining unit that determines an action of the wearer based on an interval, a pattern, and/or an output of the contact signal acquired by the contact signal acquiring unit; and an action signal output unit that outputs an action signal when a determination result of the action determining unit is determined to be a specific action.

The contact signal acquired by the contact signal acquiring unit is a voltage value. the contact signal may be a current value, another electric signal, or the like detected by the contact detection sensor which detects the contact of the wearer, but is desirably a voltage value particularly. By acquiring the voltage value as the contact signal, it is possible to acquire a change in the voltage value depending on how the wearer contacts (touch gently, touch firmly, etc.), and as a result, it is possible to detect the contact of the wearer in more detail.

The action determining unit can be configured to execute logic for determining the action of the wearer based on the contact signal acquired by the contact signal acquiring unit. Particularly, in the case of determining whether or not the action of the wearer is removing a medical catheter or removing a bandage, a covering material, a skin bonding tape, or the like (that is, "specific action"), it is desirable to determine by the interval of the contact signal. For example, when an acquisition interval between a first contact signal and a second contact signal is 0.25 seconds or more and 1.5 seconds or less, preferably 0.5 seconds or more and 1.5 seconds or less, it is determined that the specific action is performed, whereby false alarms can be greatly reduced.

That is, for a wearer such as a patient wearing a medical catheter, a bandage, a covering material, a skin bonding tape, or the like, self-removal may occur when the wearer continues to touch these attachments due to itching or the like. On the other hand, the wearer may also touch these attachments through other actions. For example, a wearer of a nasogastric feeding tube may touch the nasogastric feeding tube through an action such as preparing a pillow, sleeping in a supine position, turning over, placing the hands behind the head in a supine position, sleeping with the hands or arms under the face, scratching the back, scratching the head, wiping saliva, wiping a nasal discharge, wiping the hands and face with a wet towel, yawning, sneezing, covering the mouth with the upper arm, resting the chin in the hands on a table, or lying face down on a table with the hands or arms under the face. Therefore, it has been found that the specific action can be identified by focusing on the interval of the contact signal from the viewpoint of distinguishing the specific action from other actions, and further, when the acquisition interval between the first contact signal and the second contact signal is 0.25 seconds or more and 1.5 seconds or less, preferably 0.5 seconds or more and 1.5 seconds or less, it is determined that the specific action is performed, whereby the notification based on the contact of actions other than the specific action (that is, "false alarm") is greatly reduced.

The present disclosure provides a biological contact detection device configured using the contact motion determining module according to the present disclosure. That is, provided is a biological contact detection device including: a contact detection sensor that detects a contact of a wearer; and a contact motion determining module that predicts and determines a specific action of the wearer, wherein the contact motion determining module is the contact motion determining module according to the present disclosure.

In such a biological contact detection device, the contact detection sensor that detects the contact of the wearer may detect a pressure at the time of contact in addition to detecting an electric signal such as a current, a voltage, and a resistance. Particularly, it is desirable that the contact detection sensor includes a detection unit including one or more pairs of wirings or electrodes having different polarities. In this case, it is desirable that the detection unit is configured such that the one or more pairs of wirings or electrodes having different polarities are arranged adjacent to each other or facing each other at an interval at which at least one of a current, a voltage, and an electric resistance changes due to contact of a biological. With such a configuration, it is possible to reliably detect the contact of the wearer, and thus, it is possible to eliminate erroneous detection (excessive detection) which is detected only by approaching, and it is possible to reliably detect even a contact in which only slight pressure acts, and thus, it is possible to solve a problem of alarm failure.

Furthermore, it is desirable that the detection unit includes a sheet-like base member and one or more pairs of wirings or electrodes having different polarities provided on one surface or both surfaces of the base member and the wirings or the electrodes exist at least at an edge portion of the sheet-like base member. In an action of peeling off the contact detection sensor, since the contact detection sensor is peeled off by holding at least the edge portion, the contact of the wearer can be reliably detected by detecting the contact of the biological at the edge portion.

The present disclosure provides an action monitoring method for monitoring a specific action of a wearer. The present disclosure provides the action monitoring method including: providing a medical holding device in a target region of the wearer where a contact of a biological is detected, while installing a contact detection sensor for detecting the contact of the biological on the medical holding device; and acquiring a contact signal from the contact detection sensor to determine a specific action of the wearer based on an interval, a pattern, and/or an output of the contact signal which has been acquired. In the action monitoring method, it is desirable that the determination of the specific action of the wearer is performed by the contact motion determining module according to the present disclosure.

### Advantageous Effects of Invention

The contact motion determining module according to the present disclosure includes a contact signal acquiring unit that acquires a contact signal from the contact detection sensor that detects the contact of the wearer, an action determining unit that determines an action of the wearer based on an interval, a pattern, and/or an output of the contact signal acquired by the contact signal acquiring unit, and an action signal output unit that outputs an action signal when a determination result of the action determining unit is determined to be a specific action. Therefore, the action determining unit can quantitatively determine whether or not there is an action of removing a medical catheter or removing a bandage, a covering material, a skin bonding tape, or the like, and can be a contact motion determining module capable of eliminating notification and the like (that is, "false alarm") at the time of other actions, and a biological contact detection device using the contact motion determining module.

Thus, it is possible to provide a contact motion determining module capable of predicting and detecting an action of removing a medical catheter or removing a bandage, a covering material, a skin bonding tape, or the like by a wearer himself/herself such as a patient with an arithmetic logic of the action determining unit, and a biological contact detection device using the contact motion determining module.

### Brief Description of Drawings

Fig. 1 is a block diagram illustrating a contact motion determining module according to the present embodiment.
Fig. 2 is a perspective view illustrating an embodiment of a contact detection sensor.
Fig. 3 is a configuration diagram of a biological contact detection device.
Fig. 4 is a perspective view illustrating a use state of the biological contact detection device.
Fig. 5 is a sequence diagram of the biological contact detection device which is configured using the contact motion determining module.
Fig. 6 is a graph showing a result of Experiment 2.
Fig. 7 is a voltage waveform diagram due to a subject action.
Fig. 8 is an ROC curve (Receiver Operatorating Characteristic curve).
Fig. 9 is a graph showing a result of Experiment 3.

### Description of Embodiments

Hereinafter, a contact motion determining module 50 according to the present embodiment and a biological contact detection device 60 using the contact motion determining module 50 will be specifically described with reference to the drawings. Particularly, in the present embodiment, a contact detection sensor 10 that detects a change in voltage value at a time of contact of a wearer is used.

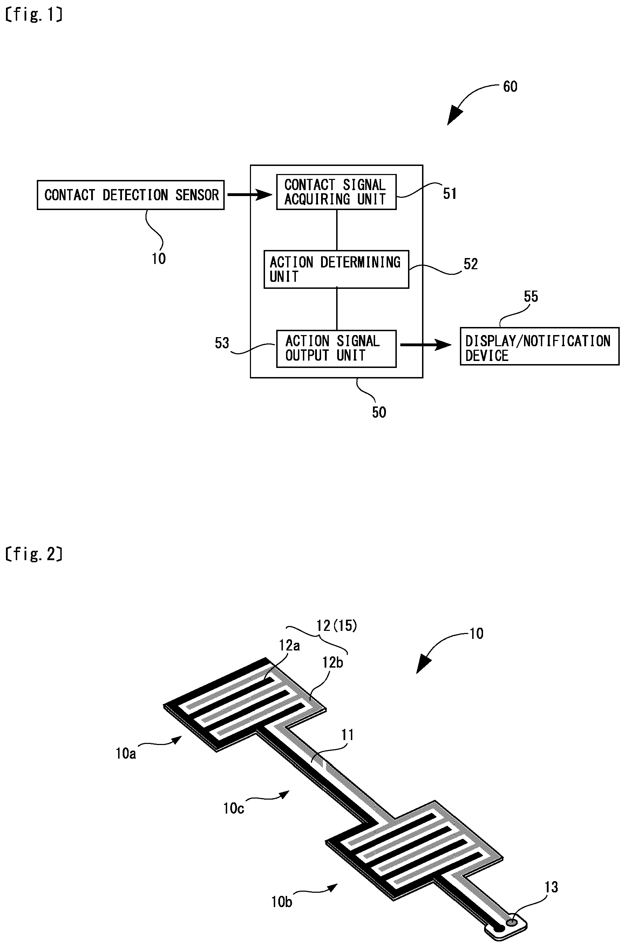

Fig. 1 is a block diagram illustrating the contact motion determining module 50 according to the present embodiment. As illustrated in Fig. 1, the contact motion determining module 50 includes a contact signal acquiring unit 51 that acquires a contact signal from the contact detection sensor 10 that detects the contact of the wearer, an action determining unit 52 that determines an action of the wearer based on an interval, a pattern, and/or an output of the contact signal acquired by the contact signal acquiring unit 51, and an action signal output unit 53 that outputs an action signal when a determination result of the action determining unit 52 is determined to be a specific action. These may be formed as a module accommodated in one housing, or may be formed with any of them accommodated in another housing. The action determining unit 52 can be formed using an arithmetic processing device of a computer, and can be formed to execute a program for determining the action of the wearer. In addition, the contact motion determining module 50 can be formed with an auxiliary storage device or a main storage device that records the program.

Particularly, in a case where the contact detection sensor 10 is configured to detect a voltage value due to the contact of the wearer, the contact signal acquiring unit 51 can be an input terminal to which the voltage signal is input. The action determining unit 52 includes a calculation unit that determines whether or not the action is removing a medical catheter or removing a bandage, a covering material, a skin bonding tape, or the like (that is, "specific action") based on the interval of the voltage signal. The action signal output unit 53 is configured to output (transmit) an action signal to an external device (such as a display/notification device 55) via a network in addition to being configured to generate voice and light. Particularly, when an action signal is output (transmitted) via a network, it is desirable to configure such that identification information unique to the contact motion determining module 50 that outputs the action signal is also output (transmitted). This is to make it possible to identify which contact motion determining module 50 the signal comes from.

Fig. 2 is a perspective view illustrating an embodiment of the contact detection sensor 10 that detects the contact of the wearer. Particularly, the contact detection sensor 10 according to the present embodiment has a structure suitable for detecting or monitoring a removal action of a nasogastric feeding tube, and is configured to be energized when a biological comes into contact with the contact detection sensor 10.

That is, the contact detection sensor 10 includes a sheet-like base member 11 and a detection unit 15 made of a conductive material provided on one surface or both surfaces of the base member 11. In the present embodiment, the base member 11 is formed using a PET sheet having a thickness of 0.075 mm, and is formed of a rectangular nose portion attachment portion 10a, a rectangular cheek portion attachment portion 10b, and a bridging portion 10c connecting the nose portion attachment portion 10a and the cheek portion attachment portion 10b to each other. Here, the base member 11 can also be formed using another resin material, and can also be formed using fabric. In addition, the shape of the base member 11 can be appropriately changed according to, for example, a site where the base member 11 is installed, and for example, the base member 11 can be formed in various shapes such as a rectangular shape, a circular shape, and an elliptical shape in addition to being formed in a belt shape and being installed in a manner of winding a target site. Furthermore, the base member 11 has a planar shape, and can also be formed in a three-dimensional shape such as a truncated cone shape.

The base member 11 may be formed using a resin sheet having stretchability and flexibility as long as a wiring or an electrode 12 constituting the detection unit 15 is not disconnected. This is to follow the movement of a user such as a patient wearing the device, and to alleviate discomfort at the time of wearing.

The detection unit 15 provided on the sheet-like base member 11 can be formed of various conductive materials, and carbon is used in the present embodiment. The detection unit 15 can include one or more pairs of wirings or electrodes 12 having different polarities. In the present embodiment, the detection unit 15 is formed by a pair of electrodes 12 including an anode 12a and a cathode 12b. Here, the detection unit 15 may be configured with a plurality of electrodes 12 including the anode 12a and the cathode 12b, and the number of electrodes 12 of one polarity may be greater than the number of electrodes 12 of the other polarity. Particularly, in the present embodiment, the electrodes 12 are formed such that the anode 12a and the cathode 12b formed in a belt shape are alternately adjacent to each other, and each of the anode 12a and the cathode 12b is formed in a comb tooth shape. By providing the electrodes 12 in such a comb tooth shape, the sensitivity at the time of biological contact can be uniformly detected in any region.

In addition, it is desirable that the detection unit 15 is provided in a manner of existing at least at an edge portion of the sheet-like base member 11, desirably in a region of less than 3 mm from the edge portion, and more desirably in a region of 1 mm or less from the edge portion. In many cases, a wearer who intends to peel the contact detection sensor 10 grasps and peels the edge portion of the contact detection sensor 10, and thus, the wirings or the electrodes 12 constituting the detection unit 15 are provided at the edge portion of the sheet-like base member 11, whereby the possibility of detecting the specific action can be increased. Furthermore, in the detection unit, it is desirable to form the wirings or the electrodes 12 formed at the edge portion of the sheet-like base member 11 in a manner of being denser than the wirings or the electrodes 12 formed in other regions. For example, it is desirable to form the wirings or the electrodes 12 formed at the edge portion in a manner of being thinner than the wirings or the electrodes 12 formed in other regions and to narrow the interval between the anode 12a and the cathode 12b.

The wirings or the electrodes 12 constituting the detection unit 15 are provided by exposing a conductive material. By exposing the conductive material, at least one of the current, the voltage, and the electric resistance is changed by the contact of the biological, and the contact of the biological can be detected based on the change amount and the change time thereof. Therefore, it is desirable that the wirings or the electrodes 12 having different polarities are formed with a thickness and an interval at which at least one of a current, a voltage, and an electric resistance can be changed by contact of the biological. In the present embodiment, the detection unit 15 is formed to detect a self-removal action of a nasogastric feeding tube in a manner that the anode 12a and the cathode 12b formed in a belt shape are alternately adjacent to each other, and each of the anode 12a and the cathode 12b has a width of 0.1 mm to 12 mm and is formed to be separated from each other at an interval of 2 mm to 12 mm. Since the intentional self-removal action of the nasogastric feeding tube is performed by fingers, the wirings or the electrodes 12 having different polarities are formed with a width and an interval capable of detecting contact of fingers.

Particularly, in the contact detection sensor 10 according to the present embodiment, the electrodes 12 (the anode 12a and the cathode 12b) can be formed using a metallic material such as gold, silver, copper, or a conductive material (such as carbon). Particularly, in the case of using a material that can be printed, for example, carbon, the electrodes 12 can be formed by printing on the base member 11, and as a result, a manufacturing process and equipment can be simplified, and manufacturing can be performed at a low cost. In addition, in a case where the electrodes 12 are formed of metal, the electrodes can be stretched due to the spreadability of the metal itself, and thus, in a case where the base member 11 having flexibility is used, the electrodes 12 can be made difficult to be cut.

In the present embodiment, the detection unit 15 is disposed without electrically connecting a pair of the electrodes 12 having different polarities. As a result, when power is supplied to each of the electrodes 12, the electrodes 12 are short-circuited by contact of the biological, and electricity can flow. Then, the presence or absence of contact of the biological can be monitored by acquiring a voltage value when electricity flows.

Since the detection unit 15 made of the conductive material is provided on the sheet-like base member 11, the contact detection sensor 10 formed as described above can be directly installed on the biological or provided on a medical holding device such as a bandage or an adhesive tape provided on the biological. That is, since it is unnecessary to select an applicable place, the contact detection sensor 10 has high versatility. Particularly, the conductive material is formed in a manner that the wirings or the electrodes 12 are exposed, electricity caused by contact with the conductive material flows, and contact of the biological can be detected based on a change in voltage, and thus, an action (contact of the biological) at the time of self-removal can be more accurately detected and prevented as compared with a case where a pressure at the time of contact is detected.

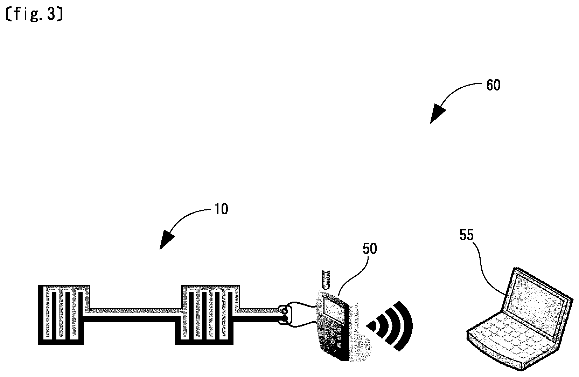

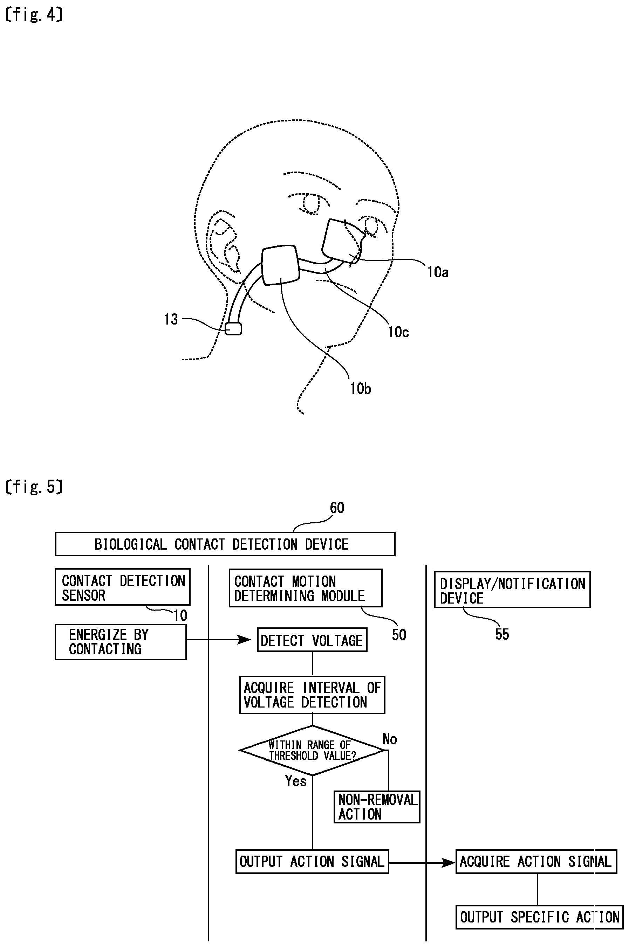

Fig. 3 is a configuration diagram of the biological contact detection device 60 including the contact detection sensor 10 and the contact motion determining module 50, Fig. 4 is a perspective view illustrating a use state of the biological contact detection device 60 including the contact detection sensor 10 and the contact motion determining module 50, and Fig. 5 is a sequence diagram of the biological contact detection device 60 which is configured using the contact motion determining module 50.

The biological contact detection device 60 includes the contact detection sensor 10 and the contact motion determining module 50 that determines presence or absence of biological contact based on a change in voltage acquired from the detection unit 15 of the contact detection sensor 10. In the present embodiment, a connection terminal 13 for connecting the contact motion determining module 50 is provided, and the contact motion determining module 50 can be connected to the connection terminal 13.

The contact motion determining module 50 can be configured with a determination circuit (an integrated circuit or the like) for determining the presence or absence of biological contact based on the change amount and the change time of the voltage acquired by the detection unit 15. Particularly, certain threshold values can be set for the change amount and the change time of the voltage, whereby it is possible to avoid a false alarm at the time of simple contact that is not intended to remove a medical catheter such as a nasogastric feeding tube or remove a bandage, a covering material, a skin bonding tape, or the like. That is, in the present embodiment, the detection interval of the voltage value is set as the threshold value, and when the detection interval is within the range of the threshold value, it can be determined that the wearer is performing an action of removing a medical catheter or an action of removing a bandage, a covering material, a skin bonding tape, or the like (that is, "specific action"). Such a threshold value can be adjusted with respect to the change time of the resistance value or the like according to a purpose of use (for example, drip self-removal prevention, endotracheal intubation tube, bladder indwelling catheter, drainage drain, arterial line, central venous catheter, nasal feeding tube, wound portion, diaper, and the like).

When the contact motion determining module 50 determines that the contact is for removing a medical catheter or removing a bandage, a covering material, or a skin bonding tape, the action signal output unit 53 outputs an action signal. Such an action signal can be output to a nurse call or a linked terminal (such as the display/notification device 55), and can also be notified (output) by an alarm, a warning light, or the like. Furthermore, the action signal output unit 53 can be equipped with a wireless output unit that wirelessly outputs the action signal. With the wireless output unit, the biological contact detection device 60 can be used wirelessly, and thus, it is possible to notify a biological contact signal even if a nurse, a caregiver, or the like is not at the bedside, and it is possible not to restrict the patient's action when the biological contact detection device 60 is used.

### [Example 1]

In this experiment, an action of actually removing a medical catheter or an action of removing a bandage, a covering material, a skin bonding tape, or the like (that is, "specific action") was identified to output an action signal using the biological contact detection device, which is illustrated in Fig. 3 and is configured using the contact detection sensor of Fig. 2, and the accuracy of outputting the action signal, that is, the reduction of false alarms of outputting an action signal at the time of an action other than these specific actions was verified.

### [Experiment 1]

In Experiment 1, the degree of alarm failure or false alarm in a conventional biological contact detection device was confirmed. That is, as the contact detection sensor, the contact detection sensor illustrated in Fig. 2 was a contact detection sensor in which the sheet-like base member 11 was provided around the detection unit 15, that is, a contact detection sensor in which the detection unit 15 including the wirings or the electrodes was provided 3 mm or more inside from the edge portion of the sheet-like base member. In addition, a contact signal was generated without the action determining unit when the biological came into contact with the contact detection sensor and energized the contact detection sensor and the voltage increased.

A false alarm was generated in a case where an action other than the specific actions was performed when the contact detection sensor installed on the wearer was energized by the contact of the wearer and the voltage increased, and an alarm failure was generated when the contact detection sensor was not energized even though a specific action was performed and the voltage did not increase.

The contact detection sensor was provided in a manner that an investigation tube was inserted into the nostril of the wearer in a non-invasive range (0.5 cm) and the investigation tube was fixed with a medical tape. Then, the action of peeling the contact detection sensor was defined as an assumed self-removal action (that is, specific action), and the other actions were defined as non-self-removal actions. The following 15 items were set as non-self-removal actions.

### "Non-self-removal actions"

· Prepare a pillow.
· Sleep in a supine position.
· Turn over.
· Place the hands behind the head in a supine position.
· Sleep with the hands or arms under the face.
· Scratch the back.
· Scratch the head.
· Wipe saliva.
· Wipe a nasal discharge.
· Wipe the hands and face with a wet towel.
· Yawn.
· Sneeze.
· Cover the mouth with the upper arm.
· Rest the chin in the hands on a table.
· Lie face down on a table with the hands or arms under the face.

In addition, the attributes of the target person to be examined in this experiment are as follows.

### "Verification target person attributes"

| | |
|---|---|
| Occupation (persons) | 20 nurses (100.0%) |
| Median age (years) (interquartile range) | 29.5 years old (22 - 41) |
| Median years of experience (years) (interquartile range) | 7.0 years (0.1 - 17.0) |
| Presence or absence of care experience of elderly with cognitive function decline | Yes 20 persons (100.0%) |
| | No 0 person (0.0) |
| Gender (persons) | 3 males (15.0%) |
| | 17 females (85.0% |
| Median horny water content (interquartile range) First finger | 33.0 (16 - 66) |
| Second finger | 37.0 (19 - 64) |
| Palm center portion | 30.5 (18 - 54) |

For the above verification target persons, there were variations in the years of experience, but the care experience of elderly with cognitive function decline was present in all the target persons, and the reproducibility of the action was maintained.

Then, the actions of the wearer were recorded on video, and the voltage value of the contact detection sensor was measured with a contact voltage measuring device to verify the occurrence conditions of the false alarm and the alarm failure.

As a result, among 20 cases, 3 cases of alarm failure occurred, and the alarm failure rate was 15%. The main cause of the alarm failures was insufficient contact with the circuit, such as peeling of the edge of the contact detection sensor with a fingertip. Therefore, it was found to be effective to provide the detection unit 15 such as the wirings or the electrodes at least at the edge portion of the sheet-like base member as a measure against the alarm failures.

Moreover, in this experiment, among 300 cases, 70 cases of false alarm occurred, and the false alarm rate was 23.3%. As the main causes of the false alarms, 3 cases out of 70 cases were non-contact false alarms such as adhesion of moisture or oil to the circuit or twisting of the circuit, and 67 cases out of 70 cases were false alarms due to non-self-removal actions such as wiping the face with a wet towel, yawning, sneezing, or resting the chin in the hands on a table.

As a result, with respect to the non-contact false alarm, it was found to be effective to wipe the contact detection sensor and the periphery thereof with a dry cloth to eliminate twisting of the bridging portion connecting the rectangular nose portion attachment portion and cheek portion attachment portion. In addition, it was found that setting of a threshold value for outputting an action signal limited to the assumed self-removal actions was effective with respect to false alarms due to the non-self-removal actions.

### [Experiment 2]

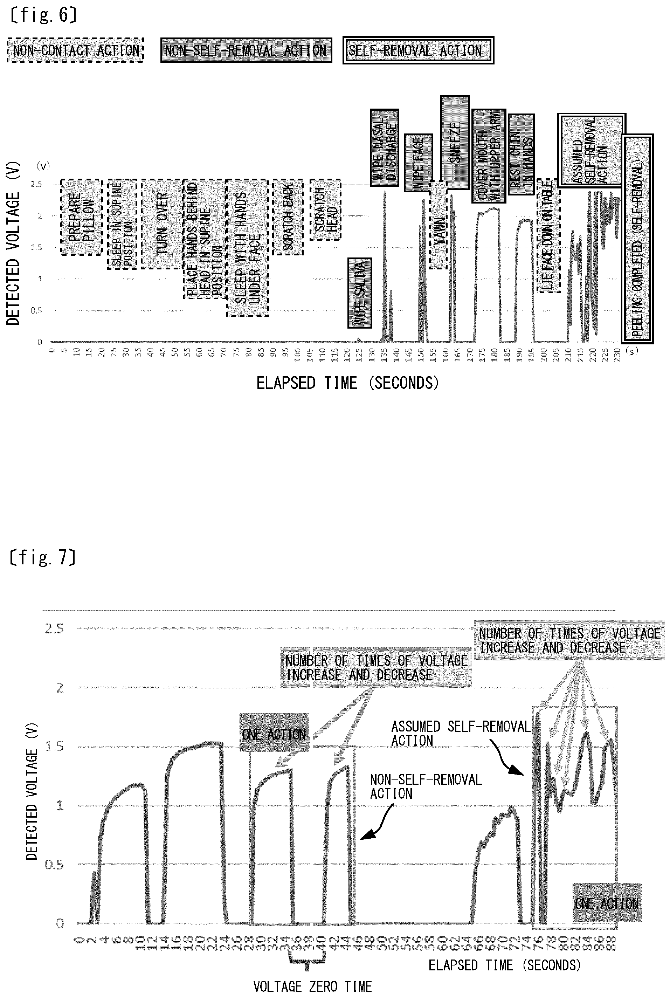

In Experiment 2, for the purpose of reducing false alarms due to the non-self-removal actions of Experiment 1, a threshold value for outputting an action signal limited to the assumed self-removal actions was examined. That is, the detected voltages of the contact detection sensor of the self-removal actions and the non-self-removal actions were measured, and a difference in voltage waveform between the self-removal actions and the non-self-removal actions was confirmed. The results are shown in Fig. 6.

In the experimental results, no voltage increase was observed in response to the actions which did not contact the contact detection sensor. On the other hand, voltage increases corresponding to each action were observed in response to the non-self-removing actions. In response to the assumed self-removal actions, a plurality of voltage peaks could be detected during the actions. Therefore, it was found to be appropriate to distinguish between the assumed self-removal actions and the non-self-removal actions based on the changes in the voltage value in response to the assumed self-removal actions and the non-self-removal actions.

That is, as shown in the voltage waveform diagram due to a subject action of Fig. 7, in the case of the non-removal actions, the number of times of voltage increase was measured twice during one action that took 16 seconds, and there was a time of 0 voltage between the measurement values for 2 seconds. On the other hand, it was found that the number of voltage increase was 6 during one operation for 12 seconds in response to the assumed self-removal actions.

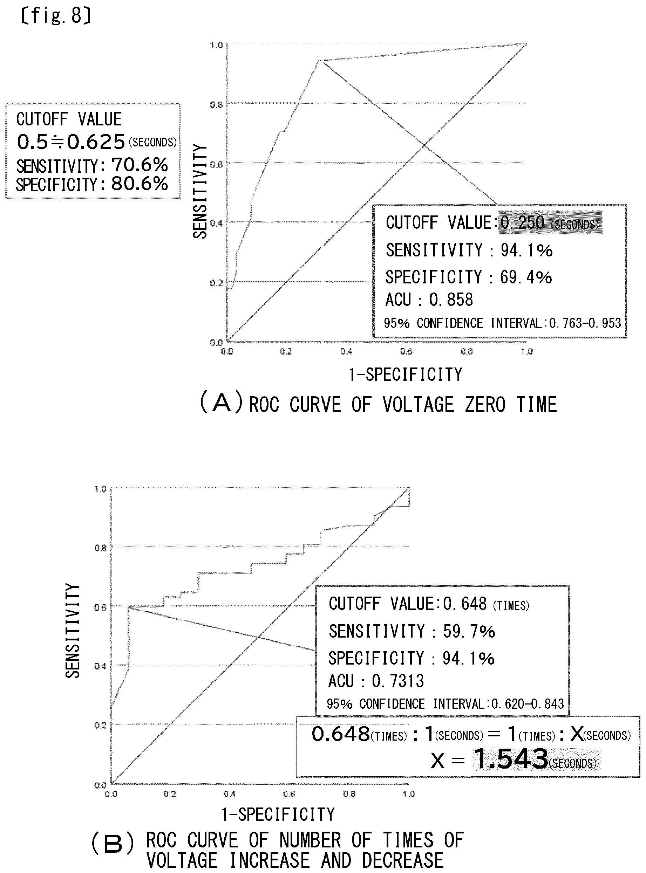

Therefore, the threshold value of the assumed self-removal actions was examined based on an ROC curve (Receiver Operatorating Characteristic curve) of the voltage zero time illustrated in Fig. 8(A) and an ROC curve of the number of times of voltage increase and decrease illustrated in Fig. 8(B).

As a result, among the non-removal actions and the assumed self-removal actions, it was found to be appropriate to determine that an action was an assumed self-removal action when there was a first voltage reaction based on the action and there was a next voltage reaction in 0.250 seconds or more and 1.543 seconds or less after the voltage became zero. Here, in order to reduce consumption of a battery in a device used for measuring a voltage value, for example, a contact voltage measuring device, it is desirable to perform measurement at 2 Hz at intervals of 0.5 seconds. Therefore, it was found that the assumed self-removal actions could be accurately detected by setting, as a threshold value, that detection of the next voltage reaction was in 0.5 seconds or more and 1.5 seconds or less after the voltage became zero.

### [Experiment 3]

In Experiment 3, as illustrated in Fig. 2, the biological contact detection device illustrated in Fig. 3 was formed using the contact detection sensor in which the detection unit 15 including the wirings or the electrodes was provided at least at the edge portion (particularly, in a range of 1 mm or less from the edge portion) of the sheet-like base member, and the accuracy of the biological contact detection device was confirmed. With the biological contact detection device of Experiment 3, in consideration of the results of Experiment 2, verification was performed by setting a threshold value of 0.5 seconds or more and 1.5 seconds or less for the time for detecting the next voltage reaction after the voltage became zero. The subjects of Experiment 3 were as follows.

### "Verification target person attributes"

| | |
|---|---|
| Occupation (persons) one nurse, one caregiver, and one physical therapist | (3 in total) |
| Median age (years) (interquartile range) | 61.0 years old (29 - 67) |
| Median years of experience (years) (interquartile range) | 8.0 years (6 - 40) |
| Presence or absence of care experience of elderly with cognitive function decline | Yes 3 persons (100.0%) |
| | No 0 person (0.0) |

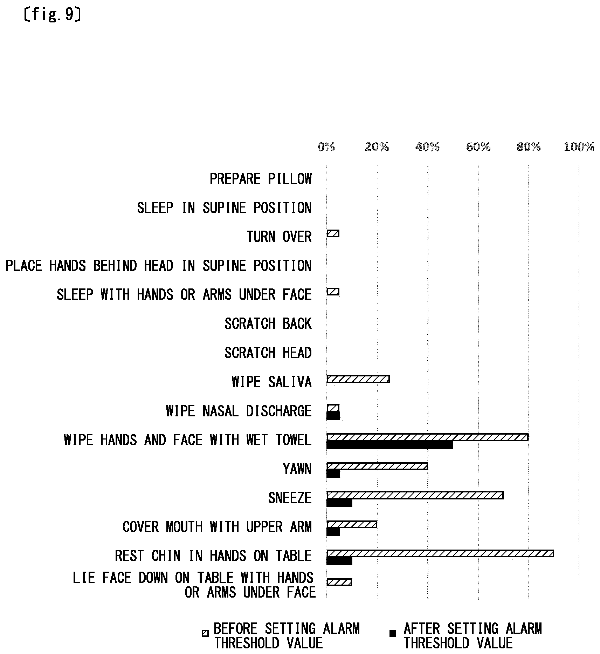

The results are shown in Fig. 9. Fig. 9 is a graph showing the results of Experiment 3 in comparison with Experiment 1. As a result, the false alarm rate, which was 23.3% in Experiment 1, was improved to 8.5%. Among the actions that caused the false alarms, the false alarm rate was high in the action of "wipe the hands and face with a wet towel", but since the action of wiping the face might destabilize a fixed portion, it was considered to be necessary to watch the wearing state of the contact detection sensor, and it was considered that there was no particular hinderance even if the action signal was output based on the action.

The false alarm rate was 2.5% for actions other than "wipe the hands and face with a wet towel". Considering that the false alarm rate of a bed-leaving sensor system in clinical practice is 52% to 70% (Yoshimura et al, 2013. Brusco et al, 2021), this false alarm rate of 2.5% is accurate enough to be used in actual clinical practice. In this experiment, both the alarm failure rate and the false alarm rate were 0.0%.

### Industrial Applicability

The contact motion determining module of the present invention and the biological contact detection device using the contact motion determining module can be used as devices for monitoring an action in which a wearer such as a patient removes a medical catheter or removes a bandage, a covering material, a skin bonding tape, or the like. In addition, by arbitrarily changing a threshold value at the time of outputting an action signal, the devices can be used as devices for assuming and monitoring a self-removal action and the like when various parts and various devices are installed.

The invention is limited by the scope of the appended claims.

### Reference Signs List

- 10: Contact detection sensor
- 10a: Nose portion attachment portion
- 10b: Cheek portion attachment portion
- 10c: Bridging portion
- 11: Base member
- 12: Electrode
- 12a: Anode
- 12b: Cathode
- 13: Connection terminal
- 15: Detection unit
- 50: Contact motion determining module
- 51: Contact signal acquiring unit
- 52: Action determining unit
- 53: Action signal output unit
- 55: Display/notification device
- 60: Biological contact detection device

## Claims

1. A contact motion determining module (50) configured to predict and determine an action related to a self-removal in which a wearer removes a medical catheter, a bandage, a covering material, or a skin bonding tape, the contact motion determining module comprising:
a contact signal acquiring unit (51) configured to acquire a contact signal from a contact detection sensor (10) configured to detect contact of the wearer;
wherein the contact signal acquired by the contact signal acquiring unit is a voltage value;
an action determining unit (52) configured to determine whether or not an action of the wearer is the action related to the self-removal based on an interval, a pattern, and/or an output of the contact signal acquired by the contact signal acquiring unit; and
an action signal output unit (53) configured to output an action signal related to the self-removal when a determination result of the action determining unit is determined to be the action related to the self-removal action, wherein
the action determining unit is configured to monitor an interval of the contact signal acquired by the contact signal acquiring unit, and the action signal output unit is configured to output the action signal when, after a first contact signal is acquired, the contact signal becomes zero and a second contact signal is acquired in 0.25 seconds or more and 1.5 seconds or less after the contact signal has become zero.

2. A biological contact detection device (60) comprising: a contact detection sensor (10) configured to detect contact of a wearer; and a contact motion determining module (50) configured to predict and determine an action related to a self-removal in which a wearer removes a medical catheter, a bandage, a covering material, or a skin bonding tape, wherein
the contact motion determining module is the contact motion determining module according to claim 1.

3. The biological contact detection device according to claim 2, wherein
the contact detection sensor is configured to include a detection unit (15) including one or more pairs of wirings or electrodes (12) having different polarities, and
the detection unit is configured in a manner that the one or more pairs of wirings or electrodes having different polarities are arranged adjacent to each other or facing each other at an interval at which at least one of a current, a voltage, and an electric resistance changes due to the contact of the wearer.

4. The biological contact detection device according to claim 2, wherein the detection unit is configured to include a base member (11) which is sheet-like and one or more pairs of wirings or electrodes (12) having different polarities provided on one surface or both surfaces of the base member, and the wirings or the electrodes exist at least at an edge portion of the base member which is sheet-like.

5. An action monitoring method for monitoring an action related to a self-removal in which a wearer removes a bandage, a covering material, or a skin bonding tape, the action monitoring method comprising:
providing a medical holding device in a target region of the wearer where contact of the wearer is to be detected, and installing, on the medical holding device, a contact detection sensor (10) for detecting the contact of the wearer; and
acquiring a contact signal from the contact detection sensor, wherein the contact signal is a voltage value, and monitoring an interval of the contact signal which has been acquired to determine that an action of the wearer is the action related to the self-removal when, after a first contact signal is acquired, the contact signal becomes zero and a second contact signal is acquired in 0.25 seconds or more and 1.5 seconds or less after the contact signal has become zero.

## Patentansprüche

1. Kontaktbewegungsbestimmungsmodul (50), das dazu konfiguriert ist, eine Handlung im Zusammenhang mit einer Selbstentfernung vorherzusagen und zu bestimmen, bei der ein Träger einen medizinischen Katheter, einen Verband, ein Abdeckmaterial oder ein Hautklebeband entfernt, wobei das Kontaktbewegungsbestimmungsmodul Folgendes umfasst:
eine Kontaktsignalerfassungseinheit (51), die dazu konfiguriert ist, ein Kontaktsignal von einem Kontaktdetektionssensor (10) zu erfassen, der dazu konfiguriert ist, einen Kontakt des Trägers zu detektieren;
wobei das durch die Kontaktsignalerfassungseinheit erfasste Kontaktsignal ein Spannungswert ist;
eine Handlungsbestimmungseinheit (52), die dazu konfiguriert ist, basierend auf einem Intervall, einem Muster und/oder einer Ausgabe des durch die Kontaktsignalerfassungseinheit erfassten Kontaktsignals zu bestimmen, ob eine Handlung des Trägers die Handlung im Zusammenhang mit der Selbstentfernung ist oder nicht; und
eine Handlungssignalausgabeeinheit (53), die dazu konfiguriert ist, ein Handlungssignal im Zusammenhang mit der Selbstentfernung auszugeben, wenn ein Bestimmungsergebnis der Handlungsbestimmungseinheit als die Handlung im Zusammenhang mit der Selbstentfernungshandlung bestimmt ist, wobei
die Handlungsbestimmungseinheit dazu konfiguriert ist, ein Intervall des durch die Kontaktsignalerfassungseinheit erfassten Kontaktsignals zu überwachen, und die Handlungssignalausgabeeinheit dazu konfiguriert ist, das Handlungssignal auszugeben, wenn, nachdem ein erstes Kontaktsignal erfasst wurde, das Kontaktsignal Null wird und ein zweites Kontaktsignal 0,25 Sekunden oder mehr und 1,5 Sekunden oder weniger erfasst wird, nachdem das Kontaktsignal Null geworden ist.

2. Bio-Kontaktdetektionsvorrichtung (60), umfassend: einen Kontaktdetektionssensor (10), der dazu konfiguriert ist, einen Kontakt eines Trägers zu detektieren; und ein Kontaktbewegungsbestimmungsmodul (50), das dazu konfiguriert ist, eine Handlung im Zusammenhang mit einer Selbstentfernung vorherzusagen und zu bestimmen, bei der ein Träger einen medizinischen Katheter, einen Verband, ein Abdeckmaterial oder ein Hautklebeband entfernt,
wobei das Kontaktbewegungsbestimmungsmodul das Kontaktbewegungsbestimmungsmodul nach Anspruch 1 ist.

3. Bio-Kontaktdetektionsvorrichtung nach Anspruch 2, wobei
der Kontaktdetektionssensor dazu konfiguriert ist, eine Detektionseinheit (15) zu enthalten, die ein oder mehrere Paare von Verdrahtungen oder Elektroden (12) mit unterschiedlichen Polaritäten enthält, und
die Detektionseinheit in einer Weise konfiguriert ist, dass das eine oder die mehreren Paare von Verdrahtungen oder Elektroden mit unterschiedlichen Polaritäten benachbart zueinander oder einander zugewandt in einem Intervall angeordnet sind, in dem sich mindestens eines von einem Strom, einer Spannung und einem elektrischen Widerstand aufgrund des Kontakts des Trägers ändert.

4. Bio-Kontaktdetektionsvorrichtung nach Anspruch 2, wobei die Detektionseinheit dazu konfiguriert ist, ein Basiselement (11), das folienartig ist, und ein oder mehrere Paare von Verdrahtungen oder Elektroden (12) mit unterschiedlichen Polaritäten, die auf einer Oberfläche oder beiden Oberflächen des Basiselements bereitgestellt sind, zu enthalten, und wobei die Verdrahtungen oder die Elektroden mindestens an einem Randabschnitt des Basiselements vorhanden sind, der folienartig ist.

5. Handlungsüberwachungsverfahren zur Überwachung einer Handlung im Zusammenhang mit einer Selbstentfernung, bei der ein Träger einen Verband, ein Abdeckmaterial oder ein Hautklebeband entfernt, wobei das Handlungsüberwachungsverfahren Folgendes umfasst:
Bereitstellen einer medizinischen Haltevorrichtung in einer Zielregion des Trägers, in der ein Kontakt des Trägers zu detektieren ist, und Installieren eines Kontaktdetektionssensors (10) an der medizinischen Haltevorrichtung zum Detektieren des Kontakts des Trägers; und
Erfassen eines Kontaktsignals von dem Kontaktdetektionssensor, wobei das Kontaktsignal ein Spannungswert ist, und Überwachen eines Intervalls des Kontaktsignals, das erfasst wurde, um zu bestimmen, dass eine Handlung des Trägers die Handlung im Zusammenhang mit der Selbstentfernung ist, wenn, nachdem ein erstes Kontaktsignal erfasst wurde, das Kontaktsignal Null wird und ein zweites Kontaktsignal 0,25 Sekunden oder mehr und 1,5 Sekunden oder weniger erfasst wird, nachdem das Kontaktsignal Null geworden ist.

## Revendications

1. Module de détermination de mouvement de contact (50) conçu pour prédire et déterminer une action liée à un retrait autonome où un porteur retire un cathéter médical, un bandage, un matériau de revêtement ou un ruban adhésif cutané, le module de détermination de mouvement de contact comprenant :
une unité d'acquisition de signal de contact (51) conçue pour acquérir un signal de contact à partir d'un capteur de détection de contact (10) conçu pour détecter un contact du porteur ;
dans lequel le signal de contact acquis par l'unité d'acquisition de signal de contact est une valeur de tension ;
une unité de détermination d'action (52) conçue pour déterminer si une action du porteur est ou non l'action liée à au retrait autonome sur la base d'un intervalle, d'un motif et/ou d'une sortie du signal de contact acquis par l'unité d'acquisition de signal de contact ; et
une unité de sortie de signal d'action (53) conçue pour émettre en sortie un signal d'action lié au retrait autonome lorsqu'un résultat de détermination de l'unité de détermination d'action est déterminé comme étant l'action liée à l'action de retrait autonome, dans lequel
l'unité de détermination d'action est conçue pour surveiller un intervalle du signal de contact acquis par l'unité d'acquisition de signal de contact et l'unité de sortie de signal d'action est conçue pour émettre en sortie le signal d'action lorsque, après qu'un premier signal de contact est acquis, le signal de contact devient nul et un second signal de contact est acquis en 0,25 seconde ou plus et 1,5 seconde ou moins après que le signal de contact est devenu nul.

2. Dispositif de détection de contact avec un corps vivant (60) comprenant : un capteur de détection de contact (10) conçu pour détecter un contact d'un porteur ; et un module de détermination de mouvement de contact (50) conçu pour prédire et déterminer une action liée à un retrait autonome où un porteur retire un cathéter médical, un bandage, un matériau de revêtement ou un ruban adhésif cutané,
dans lequel le module de détermination de mouvement de contact est le module de détermination de mouvement de contact selon la revendication 1.

3. Dispositif de détection de contact avec un corps vivant selon la revendication 2, dans lequel
le capteur de détection de contact est conçu pour comprendre une unité de détection (15) comprenant une ou plusieurs paires de fils ou d'électrodes (12) présentant des polarités différentes, et
l'unité de détection est conçue de manière à ce que les une ou plusieurs paires de fils ou d'électrodes présentant
des polarités différentes soient disposées adjacentes l'une à l'autre ou face à face l'une de l'autre à un intervalle auquel au moins l'un d'un courant, d'une tension et d'une résistance électrique change en raison du contact du porteur.

4. Dispositif de détection de contact avec un corps vivant selon la revendication 2, dans lequel l'unité de détection est conçue pour comprendre un élément de base (11) qui est semblable à une feuille et une ou plusieurs paires de fils ou d'électrodes (12) présentant des polarités différentes prévues sur une surface ou les deux surfaces de l'élément de base, et les fils ou les électrodes existent au moins au niveau d'une partie de bord de l'élément de base qui est semblable à une feuille.

5. Procédé de surveillance d'action pour surveiller une action liée à un retrait autonome où un porteur retire un bandage, un matériau de revêtement ou un ruban adhésif cutané, le procédé de surveillance d'action comprenant :
la fourniture d'un dispositif de maintien médical dans une région cible du porteur où un contact du porteur doit être détecté, et l'installation, sur le dispositif de maintien médical, d'un capteur de détection de contact (10) pour détecter le contact du porteur ; et
l'acquisition d'un signal de contact à partir du capteur de détection de contact, dans lequel le signal de contact est une valeur de tension, et la surveillance d'un intervalle du signal de contact qui a été acquis pour déterminer qu'une action du porteur est l'action liée au retrait autonome lorsque, après qu'un premier signal de contact est acquis, le signal de contact devient nul et un second signal de contact est acquis en 0,25 seconde ou plus et 1,5 seconde ou moins après que le signal de contact est devenu nul.
